# EUROPEAN PATENT APPLICATION

(11) **EP 4 019 933 A1**
(43) Date of publication of application: **29.06.2022**
(21) Application number: 21154616.3
(22) Date of filing: 01.02.2021
(51) Int. Cl.: G01N 13/00, G01N 33/15

(54) **GASTROINTESTINAL TRACT SIMULATION SYSTEM AND METHOD**

(30) Priority: 22.12.2020 EP 20216750
(71) Applicant: ProDigest, 9052 Gent (BE)
(72) Inventor: MOENS, Frédéric, 1652 Alsemberg (BE); VANDEVIJVER, Gies, 9750 Kruisem (BE); MARZORATI, Massimo, 1050 Ixelles (BE)
(74) Representative: Nederlandsch Octrooibureau

(57) **Abstract**

The present invention relates to a method of simulating the gastrointestinal dissolution and intestinal permeation of substances, comprises providing a dynamic gastrointestinal tract simulation system comprising at least two consecutive compartments of which a first compartment simulates the stomach and a second compartment simulates the duodenum, said second compartment comprising an outer vessel and an inner vessel mounted inside the outer vessel, said inner vessel having a wall comprising a grid structure and a semi-permeable or dialysis membrane mounted around said grid structure. Said gastrointestinal tract simulation system further comprising a fluid transfer system for transferring fluids into and from said at least two consecutive compartments. The method further comprises the step of introducing and dissolving a substance in fluids simulating the physiological fluids of the gastrointestinal tract present in the first compartment, transferring said fluids from the first compartment into said inner vessel, ensuring the flow of dissolved substance from said inner vessel by permeation through the semi-permeable or dialysis membrane, and removing the fluids from the said outer vessel.

## Description

### Field of the Invention

The present invention relates to a method of simulating the dynamic interplay between gastrointestinal release, dissolution, and/or digestion and subsequent intestinal absorption of substances and/or compounds, in particular pharmaceutical substances and/or compounds. The present invention further relates to a gastrointestinal tract simulation system, a compartment for such a system, and a method of operating such a system. The gastrointestinal tract simulation system can be a simulator for any monogastric animal (including humans).

### Background

The conventional SHIME is a dynamic model of the human gut comprising compartments respectively simulating the stomach, small intestine and ascending, transverse and descending colon. The stomach and small intestine compartments mimic the enzymatic and physicochemical environment by controlling pH and residence time and the dosing of a proper nutritional medium, enzymes and bile salts.
By controlling the pH, redox potential and residence times, the different colon compartments each harbor a microbial community that corresponds to that of the in vivo situation in terms of metabolic activity and community composition. As such, this system is a validated in vitro tool to perform pre-clinical research about the bidirectional interaction between orally ingested substances and the colonic microbiota activity and composition. However, this model was not designed and optimized to generate in vivo predictive pre-clinical data about the gastrointestinal disintegration, dissolution, and/or digestion and subsequent absorption of substances and more particular pharmaceutical substances and/or compounds.

A large body of new chemical entities suffer from very low aqueous solubility thereby resulting in a very low systemic exposure of the drug upon ingestion. Since only the dissolved fraction of a drug can be absorbed, pharmaceutical companies try to overcome this bioavailability-limiting factors by using several types of formulation strategies such as, among others, pH adjusting excipients, amorphous solid dispersions, surfactants, and cyclodextrins and/or dosing of the compound under fasted or fed state conditions (food effects) which have the ultimate goal to increase the amount dissolved drug in the small intestine thereby potentially increasing the percentage of the administered dose available for small intestinal absorption. In order to accelerate drug development pharmaceutical companies are more and more relying on pre-clinical data obtained through in vitro systems that are used to study the dissolution of pharmaceutical compounds. Static dissolution systems (no dynamics in concentration of tested compound in function of time, absence of dynamic pH conditions in stomach, absence of dynamic concentrations of solubilizing substances in the small intestine) can be used to obtain preliminary pre-clinical data about factors affecting the solubility of a pharmaceutical compound. Considering the highly dynamic nature of the gastrointestinal tract in terms of stomach emptying patterns (fed *versus* fasted state), fed state stomach pH conditions in function of time, and intercoupled dynamic concentrations of enzymes, bile salts, phospholipids in the small intestine in function of time, the use of simulatory systems, incorporating the dynamic interplay of all these environmental parameters could result in a more in-depth insights into the dissolution of pharmaceutical compounds along the gastrointestinal tract thereby potentially resolving research questions that could not be addressed using static systems. However, increasing the solute intraluminal small intestinal concentration of substances through specific formulation strategies or due to micellar solubilization under fed state conditions does not always result in increased absorption of the substance, a phenomenon which is known as the solubility-permeability interplay. Increasing the solubility of pharmaceutical compounds under fed state conditions or by using cyclodextrins or surfactants mostly rely on the entrapment and solubilization of the drug in bile salt micelles, surfactant micelles and cyclodextrin complexes thereby increasing the apparent solubility (total amount of detectable compound in solution) of the drug. However, to become available for absorption the drug needs to get out of these solubilizing drug structures and become molecularly dissolved. Depending on the formulation strategy and drug compound used the equilibrium between the solubilized (micelles and complexes) state and the molecularly dissolved (free compound) state can be favored towards the direction of the solubilized state, hence the increase in solute concentration will not be translated into increased absorption. Differentiating between both solute states of a drug can be performed using permeation barriers which can distinguish between drug complexes/micelles (solubilized state) and freely molecularly dissolved drugs. The use of static dissolution systems coupled with offline permeation devices could be used as a pre-clinical tool to study dissolution and permeation of a drug. However, due to the inherent dynamic nature of the dissolution of compounds in the gastrointestinal tract it is important to investigate the kinetics of the interrelated dissolution and permeation processes of a compound. Conditions in which the luminal concentration of the compound in continuously changing and the luminal compartment of the small intestine is directly coupled to the absorptive compartment. These dynamic interconnected dissolution and permeation processes are not simulated when using offline permeation tools or static coupled dissolution/permeation in vitro models. Next to this, these in vitro tools suffer from several drawbacks. In these systems the permeation of pharmaceutical compounds is tested by adding the compound to the donor side (simulating the intestinal lumen) of the device which is in contact with an acceptor solution (simulating the blood stream). In between both compartments a permeation barrier is present which only allows the molecularly dissolved fraction of the compound to permeate from the donor compartment to the acceptor compartment. Depending on the system, multiple factors can result in the absence of (sufficient) permeation in the device and the generation of biased data. Factors include:
- Precipitation of the drug in the donor compartment when using non-biorelevant aqueous buffer systems in the donor compartment.
- Insufficient concentrations of the drug in the experimental set-up and incorrect concentrations of the drug at the donor side due to the static nature of the device and the absence of biorelevant dissolution volumes in the donor compartment.
- The presence of a substantial unstirred water layer (UWL) at the donor and acceptor side of the permeation barrier due to poor hydrodynamics in the system, which will result in decreased permeation of the compound.
- Absence of pH control in the donor compartment of the permeation device which is crucial when studying compounds that have a pH-dependent solubility and hence permeability.
- The absence of biorelevant concentrations of the compound in function of time in the donor compartment due to the static nature of the device.
- The use of permeation barriers that result in a slow rate of permeation of the compound.
- A low permeation area to donor volume ratio in the device (typically < 0.5 cm⁻¹ in existing systems) resulting in slow rate of permeation and hence the absence of substantial (measurable) amounts of compound permeated during physiologically relevant time periods.
- The absence or the inability to maintain substantial sink conditions in the acceptor compartment to allow appropriate permeation.
- Non-biorelevant donor volumes in the donor compartment making it impossible to study real life dosage forms in the system.
- The inability to sample both the donor and acceptor compartment in function of time without disrupting the system.
- Adsorption of the drug to the experimental system.
- The absence of a specific geometry of the device that allows it to be incorporated into a dynamic dissolution in vitro tool.

Hence, an in vitro model that can accurately simulate the physiological conditions in the stomach and small intestine (pH and biorelevant media), and the dynamics of the gastrointestinal tract (gastric secretion, gastric emptying, dynamic stomach pH, duodenal secretion, small intestinal absorption, and small intestinal emptying) and that has an online permeation device is required to accurately simulate the dynamic gastrointestinal dissolution of a drug and its associated interconnected intestinal absorption (permeation). More specifically the online permeation compartment of the in vitro system should be optimized to overcome the drawbacks associated with the existing permeation systems (see above) and therefore should be designed in such a way that:
- It can be incorporated it into a dynamic dissolution system.
- The donor side of the permeation device is the actual dynamic small intestinal dissolution compartment.
- Allow gastric emptying and duodenal secretions enter the donor compartment and allow small intestinal emptying from the donor compartment.
- The donor compartment should contain biorelevant small intestinal volumes.
- Perform active pH control of the content of the donor compartment.
- Perform active stirring of the content of the donor and acceptor compartment.
- Have a geometry that increases the area of permeation to volume of donor compartment ratio to biorelevant levels (1.5 -2 cm⁻¹).
- Ratio of donor to acceptor fluid ratio can be adapted and optimized.
- Sink conditions can be maintained by active sink replacement in the acceptor compartment.
As such biorelevant dissolution of the compound is directly coupled to a permeation barrier that is designed in such a way that sufficient (detectable) permeation of a pharmaceutical compound during physiologically relevant time periods (biorelevant transit of dosage form) can be generated.

### Object of the Invention

An object of the invention is to provide an improved method of simulating the (dynamically interconnected) gastrointestinal dissolution and intestinal permeation of substances, preferably pharmaceutical substances. Throughout the text, unless explicitly mentioned differently, the term "substance" and "compound" are preferably used interchangeably, as is the case for the term "absorption" and "permeation". Another object of the invention is to provide an improved method of simulating the gastrointestinal dissolution and intestinal permeation of substances using a gastrointestinal tract simulation system.

### Summary of the Invention

This object is achieved by a method of simulating the gastrointestinal dissolution and intestinal permeation of a (pharmaceutical) substance according to independent claim 1.

In a first aspect, which can occur in combination with the other aspects and embodiments of the invention which are described herein, the present invention relates to a method of simulating the gastrointestinal dissolution and intestinal permeation of substances, for example, the substance may be pharmaceutical compounds, food components, chemically synthesized compounds, biological compounds, polysaccharides, proteins, peptides, amino acids, or lipids. The method comprises providing a dynamic gastrointestinal tract simulation system comprising at least two consecutive compartments of which a first compartment simulates (at least part of) the stomach and a second compartment simulates (at least part) of the small intestine, preferably the duodenum, said second compartment comprising an outer vessel and an inner vessel mounted inside the outer vessel, said inner vessel having a wall comprising a grid structure, preferably cylindrical, and a semi-permeable membrane, preferably a dialysis membrane i.e. a semi-permeable film containing various sized pores, mounted around said grid structure. The gastrointestinal tract simulation system further comprises a fluid transfer system for transferring fluids into and from said at least two compartments.

The term "semi-permeable membrane" as used herein is intended to designate a comparatively thin physical structure of such a character that certain dissolved compounds or substances with a molecular size below a certain molecular size cut off, depending on the pore size of the physical structure, can pass through it while others such as, for example, micellar and other colloidal drug structures and solid drug, having a molecular size above the specific molecular size cut off, cannot pass through it. Because of this meaning of the word semi-permeable membrane it is considered that any semi-permeable membrane can be defined as or considered as a form of a barrier which will allow passive diffusion of substances which have a size below the molecular size cut off of the membrane while substances with a greater size will be retained by the physical structure thereby preventing their passive diffusion.
The word "porous" indicates pore of any size or size range capable of retaining substances primarily because of the sizes of such substances.

The method further comprises the step of introducing the substance in simulated gastric fluids present in the first compartment, in a preferred embodiment, the method further comprises testing the concentration of total and dissolved substance in the first compartment through sampling of its content, transferring said gastric fluids from the first compartment into said inner vessel, testing the concentration of total and dissolved substance in said inner vessel through sampling of its content, and testing the flow of dissolved substance from said inner vessel by permeation through the semi-permeable membrane into the outer vessel through sampling of its content.
Advantageously, the above method allows to study the gastrointestinal dissolution and intestinal permeation of substances, in particular in oral dosage forms, in a dynamic dissolution model with a coupled and interconnected permeation setting. The above method allows to simulate the dissolution behavior of a substance (and optionally its specific formulation), the total and solute concentration of the substance in the stomach and small intestine, absorption of molecular dissolved substance over the intestinal wall, and the resulting concentration of the substance in the blood stream.
Contrary to permeation simulation systems that can only be used for offline assessment, i.e. under steady-state conditions, using a dynamic gastrointestinal tract simulation systems with coupled online permeation, allows to remove solute substance from the inner vessel, i.e. the small intestinal dissolution vessel, thereby reducing the solute substance concentration in the small intestine as occurring in vivo.
Contrary to pressure-driven processes, using a concentration-driven process, allows to more accurately simulate the permeation process in the small intestine as occurring in vivo.
Contrary to permeation systems an sich, using a system comprising at least two consecutive compartments with said second compartment having a semi-permeable membrane mounted around said cylindrical grid structure, allows to more accurately simulate the gastrointestinal tract, i.e. at least the stomach and the small intestine, by embedding the semi-permeable membrane mounted around said cylindrical grid structure in a dynamic in vitro gastrointestinal tract simulation system.

According to embodiments, the present invention relates to the method as described above, wherein the gastrointestinal tract simulation system is fully dynamic, i.e. a system that, during use, is continuously dynamic, meaning that liquid is approximately continuously pumped into the system and out of the system thereby simulating the continuous gastric secretion, gastric emptying, duodenal secretion, and/or small intestinal emptying upon ingestion of oral dosage forms as occurring in vivo. The gastrointestinal tract simulation system may be operated to continuously transfer, at predetermined rates, fluids from the first compartment into said inner vessel and fluids from the said inner vessel. The skilled person will understand that a fully dynamic system may comprise relatively short interruptions that do not influence the gastrointestinal dissolution and intestinal permeation.

In embodiments according to the invention, the step of providing a dynamic gastrointestinal tract simulation system comprises the substeps of providing an empty gastrointestinal tract simulation system comprising at least two consecutive empty compartments, and filling said empty compartments with fluids simulating the physiological fluids of the gastrointestinal tract. In particular, providing a first empty compartment and filling said empty compartments with simulated gastric fluids and providing a second empty compartment, comprising an inner vessel mounted into an outer vessel, and filling said empty inner vessel with simulated small intestinal fluids and filling said outer vessel with sink solution. Said simulated gastric fluid comprising an aqueous solution. In a more specific embodiment comprising an aqueous solution buffered at a specific pH range. In a more specific embodiment comprising pepsin as a proteolytic enzyme at a specified range. In a more specific embodiment comprising gastric lipase at a certain concentration range. In a more specific embodiment, the simulated gastric fluid contains food-derived compounds at a certain concentration range. Said simulated small intestinal fluid comprising an aqueous solution. In a more specific embodiment comprising an aqueous solution buffered at a specific pH range. In a more specific embodiment comprising bile salts and/or phospholipids at a certain concentration range. In a more specific embodiment comprising proteolytic enzymes like trypsin and/or chymotrypsin at a specified range. In a more specific embodiment comprising pancreatic lipase at a certain concentration range. In a more specific embodiment comprising pancreatic amylase at a certain concentration range. In a more specific embodiment, the simulated small intestinal fluid contains food-derived compounds at a certain concentration range. Said sink solution comprising an aqueous solution that functions as the acceptor solution of the permeation device namely the solution will accept the substances that permeate out of said inner vessel through the semi-permeable membrane into said outer vessel. In a more specific embodiment comprising an aqueous solution buffered at a specific pH range. In a more specific embodiment, a solution having a specific composition allowing to dissolve a substantial high amount of the substance tested thereby assuring that the sink solution will never get saturated with the test substances during the experimental run. In a more specific embodiment, a solution comprising surfactants (such as among other TPGS, Tween80, or SDS) above their critical micellar concentration thereby solubilizing the permeated drug in said outer vessel and preventing the substance from diffusion back into said inner vessel due to micellar entrapment of the drug in said outer vessel into surfactant micelles. In an embodiment, the pH of the simulated gastric fluid can be varied over time through active control of the pH in the first vessel through pH monitoring and pH setpoint adjustments using acid and base additions. The composition, comprising concentrations of ionic species, enzymes and food compounds, of the simulated gastric fluid can be varied over time through the dynamic interplay between gastric secretions into the first vessel and gastric emptying out of the first vessel. Next to this the pH of the simulated small intestinal fluid can be varied over time through active control of the pH. The composition, comprising concentrations of ionic species, food compounds, bile salts, and/or enzymes can be varied over time through the dynamic interplay between gastric emptying from the first compartment into the inner vessel of the second compartment, duodenal secretions into the inner vessel of the second compartment and small intestinal emptying out of the inner vessel of the second compartment.

According other embodiments, the present invention relates to the method as described above, wherein the gastrointestinal tract simulation system further comprises at least one stirring system for stirring the contents of the first compartment and at least one stirring system for stirring the content of the inner and/or outer vessel. For the first compartment, the stirring system preferably comprising a magnetic drive mounted underneath the respective vessel and provided for generating a rotating magnetic field, a permanent magnet located at the bottom of the respective vessel and provided for rotating with and amplifying said rotating magnetic field. For the compartments containing both an inner and outer vessel (small intestinal compartments) the stirring system preferably comprising a magnetic drive mounted underneath the respective outer vessel and provided for generating a rotating magnetic field, a permanent magnet located at the bottom of the outer vessel and provided for rotating with and amplifying said rotating magnetic field and a permanent magnet located at the bottom of the inner vessel and provided for rotating with and amplifying the magnetic field generated by the permeant magnet located at the bottom of the outer vessel. The gastrointestinal tract simulation system is further operated to mix the contents of the inner vessel and/or the contents of the outer vessel by stirring the contents of the respective vessel.

In a second aspect, which can occur in combination with the other aspects and embodiments of the invention which are described herein, the present invention relates to a method of simulating the gastrointestinal dissolution and intestinal permeation of a substance, wherein said substance is selected from the group consisting of drugs classified according to the Biopharmaceutics Classification System as belonging to Class I, Class II, Class III, or Class IV

Preferably, the present invention relates to the method as described above, wherein said substance is primarily absorbed through passive transcellular diffusion. More specifically said substance is selected from the group of pharmaceutical compounds belonging to Class I and Class II.

In a third aspect, which can occur in combination with the other aspects and embodiments of the invention which are described herein, the present invention relates to a method of simulating the gastrointestinal dissolution and intestinal permeation of a substance, wherein the gastrointestinal tract simulation system is further operated to transfer, at pre-determined rates:
gastric secretion fluids (simulated gastric fluid) from a first reservoir to the first compartment; simulated gastric fluids from the first compartment to the inner vessel of a second compartment;
duodenal secretion fluids (simulated small intestinal fluid for duodenum) from a second reservoir to the inner vessel of the second compartment; and simulated duodenal fluid (simulated small intestinal fluid for duodenum) from the inner vessel of the second compartment to a third reservoir.

According to further embodiments, the present invention relates to the method as described above, wherein the gastrointestinal tract simulation system is further operated to transfer, at pre-determined rates: 0%-50% fresh sink solution/min fresh sink solution from a fresh sink reservoir to the outer vessel of the second compartment, and 0%-50% waste sink solution/min waste sink solution from the outer vessel of the second compartment to a waste sink reservoir. The term "fresh sink solution" as used here is to designate the sink solution, freshly prepared, without containing any permeated drug. The term "waste sink solution" as used here is to designate the sink solution that is present in the outer vessel and contains the test substance that permeated from the inner vessel into the outer vessel.

In a fourth aspect, which can occur in combination with the other aspects and embodiments of the invention which are described herein, the present invention relates to a method of simulating the gastrointestinal dissolution and intestinal permeation of a substance, the gastrointestinal tract simulation system comprises at least three consecutive compartments of which a third compartment preferably simulates the jejunum.

According to further embodiments, the present invention relates to the method as described above, wherein the gastrointestinal tract simulation system is further operated to transfer, at pre-determined rates:
gastric secretion fluids (simulated gastric fluids) from a first reservoir to the first compartment; simulated gastric fluids from the first compartment to the inner vessel of a second compartment;
duodenal secretion fluids (simulated small intestinal fluid for duodenum) from a second reservoir to the inner vessel of the second compartment; simulated duodenal fluid (simulated small intestinal fluid for duodenum) from the inner vessel of the second compartment to the third compartment;
jejunum secretion fluids (small intestinal simulation fluid for jejunum) from a third reservoir to the third compartment; and simulated jejunum fluids (simulated intestinal fluid for jejunum) from the third compartment to a fourth reservoir. The third compartment may comprise an outer vessel and an inner vessel mounted inside the outer vessel, said inner vessel having a wall comprising a cylindrical grid structure and a semi-permeable membrane, preferably a dialysis membrane, mounted around said cylindrical grid structure.

In a fifth aspect, which can occur in combination with the other aspects and embodiments of the invention which are described herein, the present invention relates to a method of simulating the gastrointestinal dissolution and intestinal permeation of a substance, the gastrointestinal tract simulation system comprises at least four consecutive compartments of which a fourth compartment preferably simulates the ileum.

According to further embodiments, the present invention relates to the method as described above, wherein the gastrointestinal tract simulation system is further operated to transfer, at pre-determined rates:
gastric secretion fluids (simulated gastric fluid) from a first reservoir to the first compartment; simulated gastric fluids from the first compartment to the inner vessel of a second compartment;
duodenal secretion fluids (simulated small intestinal fluid for duodenum) from a second reservoir to the inner vessel of the second compartment; simulated duodenal fluid (simulated small intestinal fluid for duodenum) from the inner vessel of the second compartment to the third compartment;
jejunum secretion fluids (simulated small intestinal fluid for jejunum) from a third reservoir to the third compartment; simulated jejunum fluids (simulated small intestinal fluid for jejunum) from the inner vessel of the third compartment to the fourth compartment;
ileum secretion fluids (simulated small intestinal fluid for ileum) from a fourth reservoir to the fourth compartment; and simulated ileum fluids (simulated small intestinal fluid for ileum) from the fourth compartment to a fifth reservoir. The fourth compartment may comprise an outer vessel and an inner vessel mounted inside the outer vessel, said inner vessel having a wall comprising a cylindrical grid structure and a semi-permeable membrane, preferably a dialysis membrane, mounted around said cylindrical grid structure.

In embodiments, which can occur in combination with the other aspects and embodiments of the invention which are described herein, the present invention relates to a method of simulating the gastrointestinal dissolution and intestinal absorption of a substance, further comprising operating the gastrointestinal tract simulation system so as to control, in each of the compartments, one or more or all of the following parameters: liquid flow, temperature, pH, ionic strength, head space, stirring, pressure, liquid volume, dissolved oxygen, redox potential, each in accordance with predetermined values, ranges or trajectories.

In embodiments, which can occur in combination with the other aspects and embodiments of the invention which are described herein, the present invention relates to a method of simulating the gastrointestinal dissolution and intestinal absorption of a substance, wherein a plurality of said gastrointestinal tract simulation systems are operated simultaneously and identically, and wherein one or more of said gastrointestinal tract simulation systems contain a test sample comprising the substance and one or more of the gastrointestinal tract simulation systems do not contain a test sample.

In embodiments, which can occur in combination with the other aspects and embodiments of the invention which are described herein, the present invention relates to a method of simulating the gastrointestinal dissolution and intestinal absorption of a substance, wherein each compartment comprises a vessel having an open top surrounded by a peripheral edge portion and a lid system configured to be placed onto the peripheral edge portion and to form a tight seal between the lid system and the vessel, wherein the lid system comprises a body with a plurality of passageways extending through the body and providing access to the interior of the vessel, said plurality of passageways comprising first passageways configured for receiving fluid transfer tubes and second passageways configured for mounting at least one sensor component.

In embodiments, which can occur in combination with the other aspects and embodiments of the invention which are described herein, the present invention relates to a method of simulating the gastrointestinal dissolution and intestinal absorption of a substance, wherein the fluid transfer system preferably comprises a plurality of peristaltic pumps for pumping fluid via fluid transfer tubes into and from each compartment.

In a further aspect, which can occur in combination with the other aspects and embodiments of the invention which are described herein, the present invention relates to a gastrointestinal tract simulation system for simulating the gastrointestinal dissolution and intestinal absorption of a substance, comprising at least two consecutive compartments of which a first compartment simulates (at least part of) the stomach and a second compartment simulates (at least part) of the small intestine, preferably the duodenum, said second compartment comprising an outer vessel and an inner vessel mounted inside the outer vessel, said inner vessel having a wall comprising a cylindrical grid structure and a semi-permeable membrane, preferably a dialysis membrane, mounted around said cylindrical grid structure.

In embodiments according to the invention, which can occur in combination with the other aspects and embodiments of the invention which are described herein, said cylindrical grid structure has an interior volume of at least 25 ml, preferably at least 30 ml, more preferably at least 40 ml, even more preferably at least 50 ml. In embodiments according to the invention, which can occur in combination with the other aspects and embodiments of the invention which are described herein, the ratio of the surface area of the cylindrical grid structure to the interior volume ranges from 1 to 3 cm² per ml, preferably ranges from 1.5 to 2.5 cm² per ml, more preferably is around 2.0 cm² per ml. In embodiments according to the invention, which can occur in combination with the other aspects and embodiments of the invention which are described herein, the cylindrical grid structure may have an open area ratio of at least 50%, preferably between 60% and 80%. Preferably the cylindrical grid structure has an open top and a partially closed bottom to receive the stirring magnet. In embodiments according to the invention, which can occur in combination with the other aspects and embodiments of the invention which are described herein, the ratio of the volume of the outer vessel to the interior volume is at least 3, preferably between 5 and 15, more preferably is around 10.

### Brief description of Drawings

The invention will be explained in more detail below with reference to drawings in which illustrative embodiments thereof are shown. They are intended exclusively for illustrative purposes and not to restrict the inventive concept, which is defined by the appended claims.
Figure 1 shows in a schematic view a gastrointestinal tract simulating system according to a first embodiment of the invention;
Figure 2 shows in a schematic view a gastrointestinal tract simulating system according to a second embodiment of the invention.
Figure 3 shows a schematic view of a method of simulating the gastrointestinal dissolution and intestinal permeation of substances according to an embodiment of the invention; and
Figure 4 shows a schematic view of the vessel and lid system according to an embodiment of the invention;
Figure 5A-7B show experimental results of a study to demonstrate the capacity of the gastrointestinal tract simulating system of Figure 1 to replicate the in vivo observed complex gastrointestinal behavior upon intraluminal dilution of a cyclodextrin-based solution of itraconazole under fasted state conditions.
Figures 8-10B show experimental results of a study to demonstrate the capacity of the gastrointestinal tract simulating system of Figure 1 to replicate the in vivo observed complex gastrointestinal behavior of indinavir sulfate under fasted state conditions versus fed state conditions.

### Detailed Description of Embodiments

The present disclosure will be described with respect to particular embodiments and with reference to certain drawings but the disclosure is not limited thereto but only by the claims. The drawings described are only schematic and are non-limiting. In the drawings, the size of some of the elements may be exaggerated and not drawn on scale for illustrative purposes. The dimensions and the relative dimensions do not necessarily correspond to actual reductions to practice of the disclosure.

Furthermore, the terms first, second, third and the like in the description and in the claims, are used for distinguishing between similar elements and not necessarily for describing a sequential or chronological order. The terms are interchangeable under appropriate circumstances and the embodiments of the disclosure can operate in other sequences than described or illustrated herein.

Moreover, the terms top, bottom, over, under and the like in the description and the claims are used for descriptive purposes and not necessarily for describing relative positions. The terms so used are interchangeable under appropriate circumstances and the embodiments of the invention described herein can operate in other orientations than described or illustrated herein.

Furthermore, the various embodiments, although referred to as "preferred" are to be construed as exemplary manners in which the disclosure may be implemented rather than as limiting the scope of the disclosure.

The term "comprising", used in the claims, should not be interpreted as being restricted to the elements or steps listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a device comprising A and B" should not be limited to devices consisting only of components A and B, rather with respect to the present disclosure, the only enumerated components of the device are A and B, and further the claim should be interpreted as including equivalents of those components.

Different aspects of the present disclosure will be described more fully hereinafter with reference to the enclosed drawings. The embodiments disclosed herein can, however, be realized in many different forms and should not be construed as being limited to the aspects set forth herein.

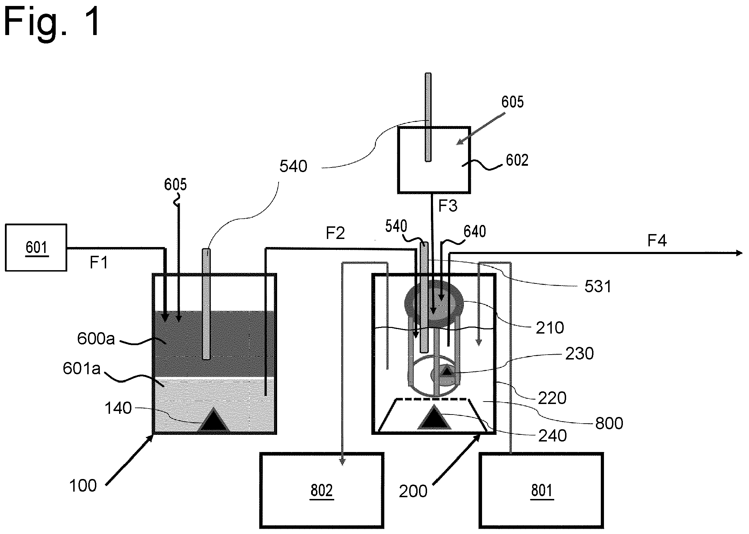

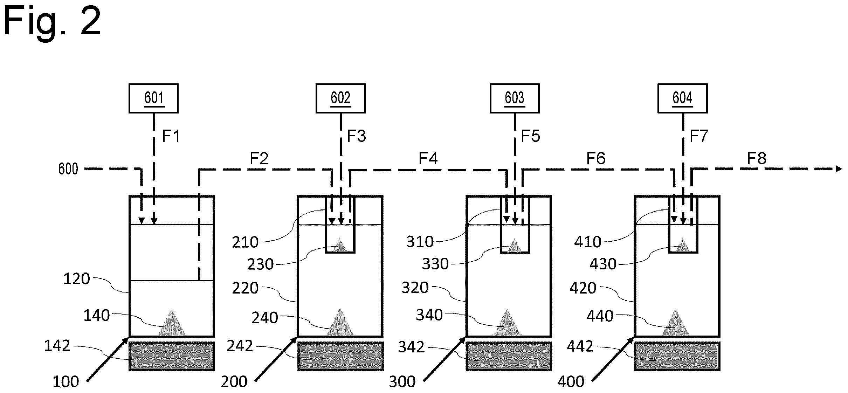

Figures 1 and 2 show a gastrointestinal tract simulating systems, comprising a number of serially arranged compartments, for example 2 to 10 serially arranged compartments, typically 3 or 4 serially arranged compartments 100, 200, 300, 400. The gastrointestinal tract simulation system further comprises a fluid transfer system for transferring fluids into and from said compartments and pH sensors to measure and control the pH values of the interior volumes of said compartments. The fluid transfer system preferably comprises a plurality of pumps, preferably peristaltic pumps (not shown), for pumping fluid via fluid transfer tubes into and from said compartments 100, 200, 300, 400.
The gastrointestinal tract simulation system comprises at least two consecutive compartments of which a first compartment 100 simulates at least part of the stomach and a second compartment 200 simulates at least part of the duodenum, said second compartment comprising an outer vessel 220 and an inner vessel 210 mounted inside the outer vessel, said inner vessel having a wall comprising a grid structure, preferably cylindrical and a semi-permeable membrane, preferably a dialysis membrane mounted around said cylindrical grid structure. The first compartment 100 is adapted to receive to simulated gastric fluids, in particular gastric secretion 601a, and pharmaceutical substances 600a according to respectively a first volume V_{fast} and a second volume V_{dose}. In this way, the inner vessel can be used to mimic the absorption of compounds in the intestines, with the membrane mimicking the blood-intestine barrier. For example, as shown in Figure 1, by introducing gastric secretion 601 in the first compartment 100 with flow rate F1, simulating gastric emptying with flow rate F2, introducing duodenal secretion 602 in the second compartment 200 with flow rate F3 and simulating duodenal emptying with flow rate F4. In addition, as shown in Figure 2, pharmaceutical substances 600 may be introduced in the first compartment and the gastrointestinal tract may be further simulated by introducing jejunal secretion 603 in the third compartment 300 with flow rate F5, simulating jejunal emptying with flow rate F6, introducing ileal secretion 604 in the fourth compartment 400 with flow rate F7 and simulating ileal emptying with flow rate F8. Additionally, as shown in Figure 1, sink 800 is transferred between the second compartment 200 and sink reservoirs, i.e. fresh sink solution 801 is transferred from a fresh sink reservoir to the outer vessel of the second compartment 200 and waste sink solution 802 is transferred from the outer vessel of the second compartment 200 to a waste sink reservoir. The term "fresh sink solution" as used here is to designate the sink solution, freshly prepared, without containing any permeated drug. The term "waste sink solution" as used here is to designate the sink solution that is present in the outer vessel and contains the test substance that permeated from the inner vessel into the outer vessel. The pH of the simulated gastric fluid can be varied over time through active control of the pH in the first vessel through pH monitoring via a pH sensor 540 and pH setpoint adjustments using acid and base additions 605.
The grid structure 210 may for example be a 3D printed part, for example in polytetrafluoroethylene, PTFE, polyamide, polypropylene, polycarbonate, acrylonitrile butadiene styrene, polylactic acid or another material suitable for 3D printing. Preferably, the grid structure 210 is a CNC machined part, for example in a solid metal (e.g. steel, aluminium, titanium, ...), a plastic (e.g. PTFE, polycarbonate, poly ether ether ketone) or polyoxymethylene. The outer vessel 220 is preferably manufactured in a transparent material to enable visual inspection of the contents of the compartment.

In embodiments, the grid structure may have an interior volume of at least 25 ml, preferably at least 30 ml, more preferably at least 40 ml, even more preferably at least 50 ml. For example, the ratio of the surface area of the grid structure to the interior volume ranges from 1 to 3 cm² per ml, preferably ranges from 1.5 to 2.5 cm² per ml, more preferably is around 2.0 cm² per ml. In particular, the grid structure may have an open area ratio of at least 50%, preferably between 60% and 80%. Furthermore, the ratio of the volume of the outer vessel to the interior volume may be at least 3, preferably between 5 and 15, more preferably is around 10. Furthermore, the geometry of the grid structure allows to insert a pH sensor making it possible to measure and actively control the pH value of the interior volume of the grid structure.

In embodiments, as shown in figures 1 and 2, a gastrointestinal tract simulation system is provided, comprising at least two compartments 100, 200, for example as described above, wherein the gastrointestinal tract simulation system further comprises at least two magnetic stirring systems for stirring the contents of at least two compartments namely the first compartment 100 and the inner vessel 210 and outer vessel 220 of the second compartment 200. The magnetic stirring system comprising a magnetic drive 142, for example a rotating electromagnetic field, mounted underneath the respective vessel 120 and provided for generating a rotating magnetic field, a first stirring element (permanent magnet) 140 located at the bottom of the vessel and provided for rotating with said rotating magnetic field. The magnetic stirring system comprising a magnetic drive 242, for example a rotating electromagnetic field, mounted underneath the respective compartment 200, a first stirring element (permanent magnet) 240 located at the bottom of the outer vessel 220 and provided for rotating with and amplifying said rotating magnetic field, and a second stirring element (permanent magnet) 230 at the bottom of the inner vessel 210 provided for being rotated by said amplified magnetic field. Stirring of the inner and outer vessels of the third 300 and fourth 400 compartment are performed as described for the inner and outer vessel of the second compartment 200. Preferably the speed of said rotating magnetic field is as high as possible while maintaining stability of the magnets. For example, this may be around 300 rpm or around 500 rpm. One reason for this is what is called the 'unstirred water layer.' This is the layer of liquid bordering (among others) the membrane, which because of fluid dynamics, resists the stirring/movement of the liquid by 'sticking' to a solid wall (the membrane). This means that layer is mixed less (or not at all) than the surrounding fluid, and thus will contain a higher concentration of the permeating substance in the outer vessel side of the membrane than the rest of liquid in the outer vessel, and vice versa on the inside vessel. This in turn means the osmotic pressure (concentration gradient) is lower than in an ideal system and lowers the permeation flux over the membrane as compared to the theoretical one. The faster the liquid moves, and the more turbulence there is, the more this phenomenon is avoided.
Another reason is the relatively high cylindrical liquid column both in the outer and especially inner vessels, compared to their diameter. As such, the stirring magnet needs to create a bigger vortex to also reach the top layers of the liquid column. For the outer vessel, this is less of an issue due to the space available allowing use of a large magnet, however in the inner vessel, space is a lot more limited, meaning only small magnets fit, which create only a small vortex, especially on low speeds. Furthermore, the stirring of the outer and inner vessels of the different compartments is necessary to allow active measurement and control of certain parameters, for example pH (see below), in said vessel.
By the provision of this magnetically driven stirring element, the need for a mechanical coupling of the stirring element through the wall of the vessel or the lid can be avoided, and as a result an air-tight sealing of the interior of the compartment can be better assured. The provision of the permanent magnet which rotates along and amplifies the generated magnetic field can ensure a good operation of the stirring element in the vessel.

In preferred embodiments, the permanent magnets 140, 240, 340, 440, 230, 330, 430 may be generally triangular prism-shaped or cylindrical-shaped. They may be freely moving elements in the respective vessel or be held in position by means of an additional positioning element (not shown).

Each compartment may comprise a vessel having an open top surrounded by a peripheral edge portion and an air-tight lid system configured to be placed onto the peripheral edge portion and to form an air-tight seal between the lid system and the vessel. This means that the lid system is configured to fit on the peripheral edge portion in such a way that, following a step of fixing the lid system on said edge portion, an air-tight seal is obtained. This can be achieved in many ways, for example involving one or more sealing rings, which are known per se to the person skilled in the art, and therefore need not be further described herein.

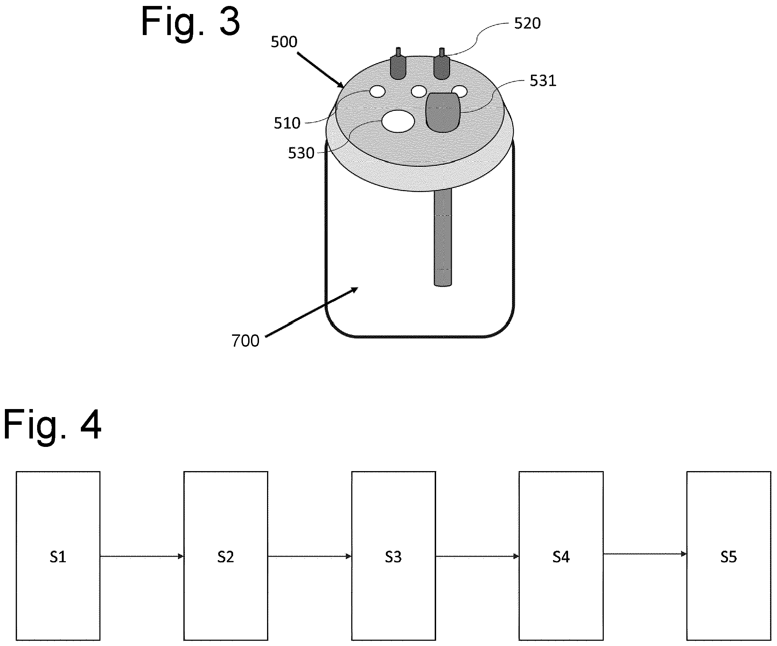

The lid system, as shown in figure 3, may comprise a body 500 with a plurality of passageways 510, 520, and/or 530 extending through the body and providing access to the interior 700 of the vessel, said plurality of passageways comprising first passageways 510 and /or 520 configured for receiving fluid transfer tubes and second passageways 530 configured for mounting at least one sensor component 531 (which may also comprise tubing). This means that the first passageways are sized and formed for receiving fluid transfer tubes (for transferring liquids and/or gases), in such a way that they preferably fit tightly around the tubes and guide them between the point of entry into the lid system and the point of exit towards the interior of the vessel, preferably so as to obtain gentle corners to avoid obstructions, and that the second passageways are configured for accommodating a sensor or at least a component of a sensor, which is in turn provided for sensing a parameter in the interior of the vessel.

Figures 4 shows a method of simulating the functioning of the human or animal gastrointestinal dissolution and intestinal absorption of pharmaceutical substances, comprising the steps of filling empty compartments of a gastrointestinal tract simulation system as described herein with fluids simulating the physiological fluids of the gastrointestinal tract and operating the gastrointestinal tract simulation system so as to control, in each of the compartments, one or more or all, but not limited to, of the following parameters: liquid flow (e.g. 0-25 ml/min), temperature (e.g. 20-50 °C), pH (e.g. ranging from 1 to 9), ionic strength (e.g. ranging from 0 to 10 M), stirring (e.g. 0-600 rpm), pressure (e.g. 0-2 bar), liquid volume (e.g. 0-1000 ml), each in accordance with pre-determined values, ranges or trajectories. Preferred ranges are: liquid flow 0-14 ml/min, temperature 35-40 °C, pH ranging from 1.5 to 8, ionic strength ranging from 0.01 to 5 M, stirring 1-500 rpm, pressure 0.1- 1.5 bar, liquid volume 30-1000 ml.
The method may comprise a first step S1 of sterilizing the interior of the gastrointestinal tract simulation system by introducing H₂O₂ gas into the empty compartments and/or by increasing the interior temperature of the compartments to a value within the range of 40 °C to 121 °C (15 psi) for a period of time within the range of 30 minutes.
The method further comprises a second step S2 of introducing a substance in the first compartment, wherein said substance is at least partially made of the compound for which the dissolution and permeation is tested.
The method further comprises operating the gastrointestinal tract simulation system to perform: a third step S3 of *'gastric emptying'* by transferring a mixture of simulated gastric fluids and the test substance from the first compartment into the inner vessel, a fourth step S4 of ensuring the flow of the dissolved substance from said inner vessel by permeation through the semi-permeable membrane, preferably a dialysis membrane, e.g. regenerated cellulose, into the outer vessel and a fifth step S5 of *'duodenal emptying'* by transferring simulated duodenal fluid out of the inner vessel of the second compartment. The method may further comprise of a multitude of additional steps (not shown) that are similar to S4 and/or S5, but pertain to subsequent compartments and/or vessels.

The gastrointestinal tract simulation system may be further operated to transfer (figure 1 and 2), at pre-determined rates: gastric secretion fluids from a first reservoir to the first compartment; simulated gastric fluids from the first compartment to the inner vessel of a second compartment; duodenal secretion fluids from a second reservoir to the inner vessel of the second compartment; simulated duodenal fluid from the inner vessel of the second compartment to the (inner vessel of the) third compartment; jejunum secretion fluids from a third reservoir to the (inner vessel of the) third compartment; simulated jejunum fluids from the inner vessel of the third compartment to the (inner vessel of the) fourth compartment; ileum secretion fluids from a fourth reservoir to the (inner vessel of the) fourth compartment; and simulated ileum fluids from the (inner vessel of the) fourth compartment to a fifth reservoir.

In embodiments, a plurality of said gastrointestinal tract simulation systems are operated simultaneously and identically, and wherein one or more of said gastrointestinal tract simulation systems contain a substance and one or more of the gastrointestinal tract simulation systems do not contain a test sample.

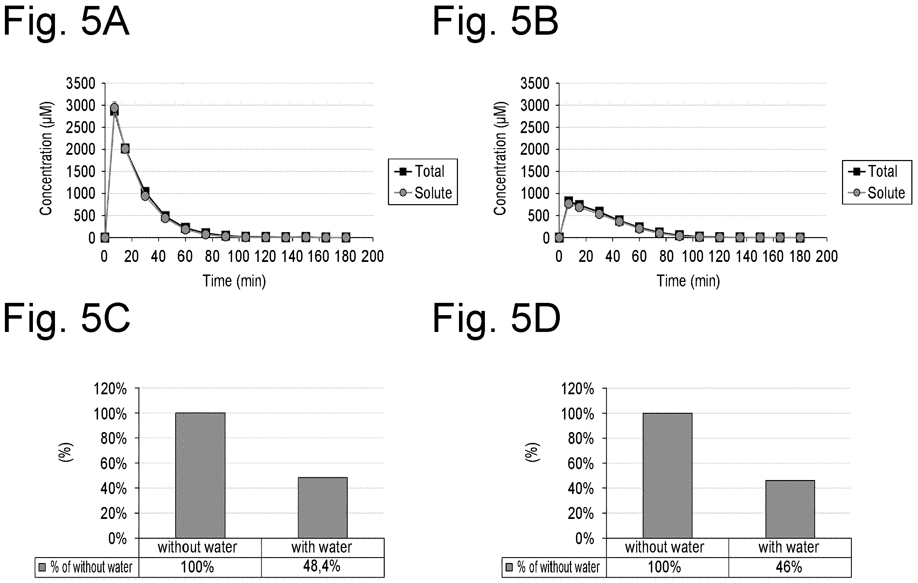

Figures 5A-7B are a series of graphs showing the results of an experiment, simulating fasted state conditions, in which tests are performed in an experimental setup as shown in figure 1. Before the start of the experiments, 55 mL of simulated gastric juice (V_{fast} = 55 mL) was added to the first compartment, to simulate the rest volume of the stomach under fasted conditions, 30 mL of simulated duodenal juice (V_{duo} = 30 mL) was added to the inner vessel of the second compartment, and 300 mL of sink solution (Vₛᵢₙₖ = 300 mL) was added to the outer vessel of the second compartment.
At the start of the first experimental run, 20 mL of Sporanox^{®} solution (corresponding to a dose of 200 mg itraconazole) was added to the stomach (without water) (V_{dose_1} = 20 mL) whereas at the start of the second run, 20 mL of Sporanox solution was added together with 240 mL of water to the first compartment (with water) (V_{dose_2} = 260 mL). During the experimental run the dynamics of the GIT were simulated through gastric secretion (flow rate F1 = 2,5 mL/min) of simulated gastric fluid into the stomach, gastric emptying (flow rate F2; combination of mono-exponential emptying of the dose and emptying of the added gastric secretion) from stomach to the inside of the inner vessel, duodenal secretion (flow rate F3= 2,5 mL/min) from an exterior reservoir inside the inner vessel and duodenal emptying (flow rate F4 = F2 + F3) out of the inner vessel. As such, the volume of the stomach decreased monoexponentially in function of time whereas the volume of the inner vessel and the outer vessel remained constant. The pH of the content of the stomach was automatically controlled at a setpoint of pH 1,6. The pH of the duodenum (inner vessel) was kept constant at a value of 6,5 through active pH control and the secretion of duodenal fluids in the inner vessel. Active pH control was performed on the duodenal secretion, making it possible to change the buffering strength of the duodenal secretions in function of time.
The content of the stomach reactor was homogenized through stirring at 300 rpm. The content of the inner and outer vessel was homogenized through stirring at 500 rpm. The temperature of the respective vessels was controlled at 37°C.
Each experimental run was followed for 180 min and samples were taken from the first compartment, the inner vessel and the outer vessel after 7, 15, 30, 45, 60, 75, 90, 105, 120, 135, 150, 165, and 180 min. In the samples taken from the stomach and duodenum the total and solute concentration of itraconazole was determined. In the samples taken from the sink (outer vessel) the solute concentration of itraconazole was determined. Itraconazole concentrations were determined through UHPLC chromatography coupled to UV detection. All experiments were performed in biological triplicate.

In the experiment, the capacity of the experimental setup to replicate the in vivo observed complex gastrointestinal behavior of itraconazole solution is studied. Itraconazole is a weak basic drug (pKa 2 and 3,7) with a very low aqueous solubility thereby resulting in low small intestinal solute concentrations of the drug and hence low systemic bioavailability. To increase its solubility in the duodenum itraconazole is commercially available as a cyclodextrin-based formulation (Sporanox^{®}, Janssen, Beerse, Belgium). In this formulation itraconazole is solubilized by means of 40% 2-hydroxypropyl-β-cyclodextrin.

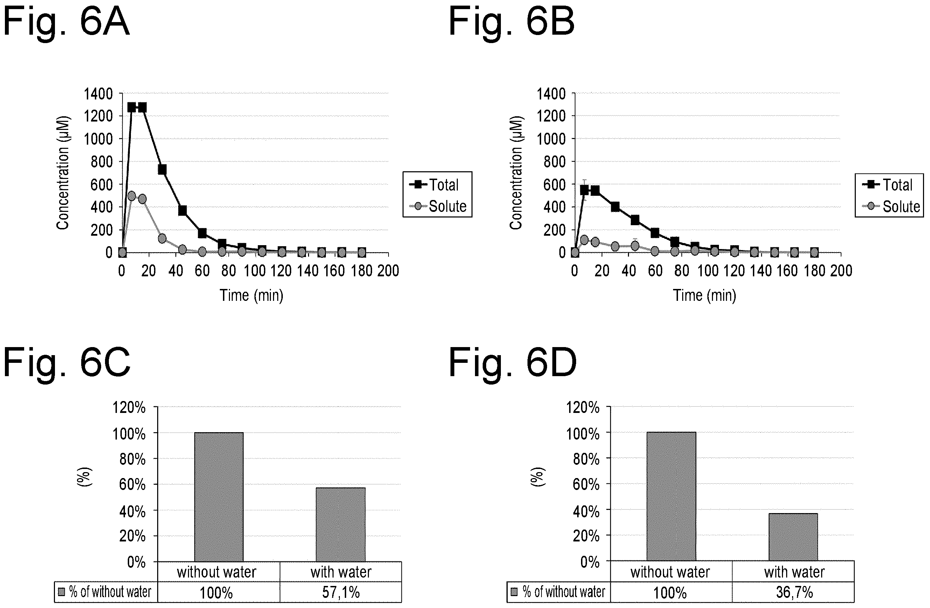

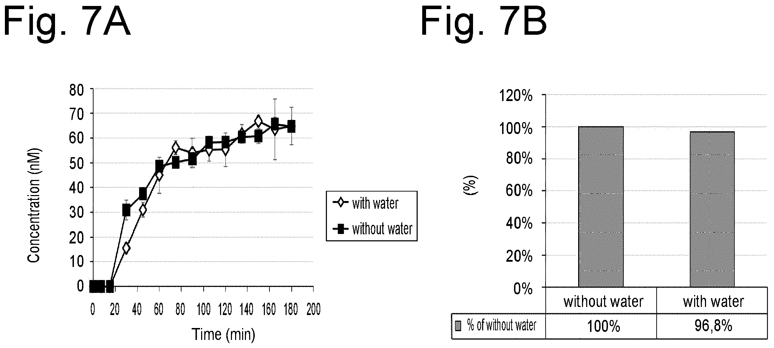

The result show that ingestion of 20 mL Sporanox solution without the concomitant administration of water resulted in high total and solute concentrations of itraconazole in the stomach. Throughout the experiment, itraconazole was completely dissolved in the stomach as was demonstrated by the equal concentrations of total and solute drug present. The complete dissolution of itraconazole in the stomach resulted from the solubilizing effect of the cyclodextrins in combination with the low pH value of the stomach which is needed for weak basic drugs to become dissolved, as shown in Figure 5A. Co-ingestion of 20 mL Sporanox solution together with 240 mL water resulted in dilution of the administered dose as was demonstrated by the lower total and solute concentration of itraconazole present under these conditions. Co-ingestion of water did not result in precipitation of itraconazole as was evident from the equal concentrations of total and solute itraconazole present in the stomach, as shown in Figure 5B.
In both conditions, the concentrations of itraconazole decreased in function of time and this due to dilution with gastric secretions and removal of the compound from the stomach through gastric emptying. Calculation of the area under the curve for both the total, as shown in Figure 5C, and, as shown in Figure 5D, concentrations of itraconazole in the stomach revealed that both co-ingestion of water decreased the area of the curve of bot the total and solute concentrations towards the same extent. Hence, it can be concluded that co-administration of water only resulted in a dilution effect of the compound and did not impact the dissolution and/or solubilization of itraconazole in the stomach. The observed time-dependent behavior of itraconazole in the stomach compartment was fully in line with the behavior observed during the in vivo studies with itraconazole (Berben et al., 2017).
Gastric emptying of the contents of the stomach into the duodenum resulted in a sharp increase in the total concentration of itraconazole in the lumen of the duodenum. Following this initial sharp increase, the total concentration of itraconazole decreased due to dilution of the content of the duodenum with duodenal secretions and removal of the drug substance through duodenal emptying. During the experiments without the addition of water, itraconazole entered the duodenum resulting in the precipitation of a fraction of the administered dose as was demonstrated by the lower concentrations of solute itraconazole as compared to the total concentration of itraconazole, as shown in Figure 6A. This precipitation was due to pH shift that occurred when the compound was transferred to the acidic environment of the stomach (pH 1.6) to the more neutral environment of the duodenum (pH 6.5). The duodenal pH was substantially higher than the pKa value of itraconazole (2 and 3.7) thereby explaining the duodenal precipitation. A remarkable difference was observed in the duodenal behavior of itraconazole between the experiments with and without co-administered water. High concentrations of solute itraconazole were present in the duodenum during the experiments without water (Figure 2A). Co-administration of water together with itraconazole resulted in substantial lower concentrations of solute itraconazole in the duodenum, as shown in Figure 6B.
As such, it was evident that the observed similarity in stomach dilution between both conditions was not translated in the same dissolution and/or solubilization behavior in the duodenum. It can be anticipated that the lower concentrations of solute itraconazole during the experiments with water were due to the dilution of the compound in the stomach. Indeed, determination of the area under the curve of the total concentration of itraconazole in the duodenum revealed that the total concentration of itraconazole in the duodenum was decreased with 32,1 % due to dilution with water, as shown in Figure 6C. As such, when the same dissolution and/or solubilization of itraconazole occurred in both conditions the same decrease in the area under the curve for the solute concentration should be observed. Co-administration of water resulted in a decrease in the area under the cure of the solute concentration with 56,1 % which is higher as would be expected by a pure dilution effect, as shown in Figure 6D. These results clearly demonstrated that the intraluminal dilution of the Sporanox solution with water resulted in an additional 20,4% of duodenal precipitation of itraconazole. This was attributed to the dilution of the cyclodextrins in the formulation resulting in a lower solubilizing capacity of itraconazole in the duodenum and hence increased precipitation of the compound. Furthermore, this also demonstrated that the increased duodenal concentrations of solute itraconazole in the run without water was due to the solubilization of itraconazole by cyclodextrin inclusion complexes. Hence co-administration of water had a devastating effect on the concentration of solute itraconazole in the duodenum, 2,28-fold decrease as compared to without water, and could potentially impact the final bioavailability of the compound. The data generated for the duodenum in the system according to the invention were fully in line with the observed data during the in vivo studies with itraconazole (Berben et al., 2017).
The system according to the invention allowed to study the dynamically interconnected dissolution and permeation of itraconazole during transit through the gastro-intestinal tract. Whereas co-administration with water resulted in devastating decrease in the concentration of solute itraconazole, the driving force for permeation, the permeation data generated during the experiments revealed the absence of a substantial effect on the permeated fraction of itraconazole in between both conditions, as shown in Figure 7A. Faster permeation occurred during the initial 60 min of the experiments without added water due to the high peak concentrations observed in the duodenum. However, this effect was only minor as compared to the difference in between solute itraconazole concentrations in the duodenum. For both conditions, the amount of permeated itraconazole reached the same plateau concentrations, as shown in Figure 7A. Determination of the area under the curve of the permeated compound revealed the absence of a substantial difference between the bioavailability of itraconazole when taken with or without water, as shown in Figure 7B.
Hence these data suggest the existence of solubility-permeability interplay for itraconazole formulated with cyclodextrins. The increased concentration of solute itraconazole in the duodenum without water was not translated into an increased absorption of the compound indicating that the affinity of itraconazole for the cyclodextrin inclusion complexes was too high to generate substantial concentrations of free molecularly dissolved itraconazole available for permeation. Intraluminal dilution of itraconazole and cyclodextrins due to water ingestion resulted most probably in the absence of higher order cyclodextrin complexes for which the compound has high affinity, thereby decreasing the solubilization of the compound but generating the same concentrations of molecularly dissolved drug available for permeation. These data were fully in line with the data generated during the in vivo study (Berben et al., 2017).

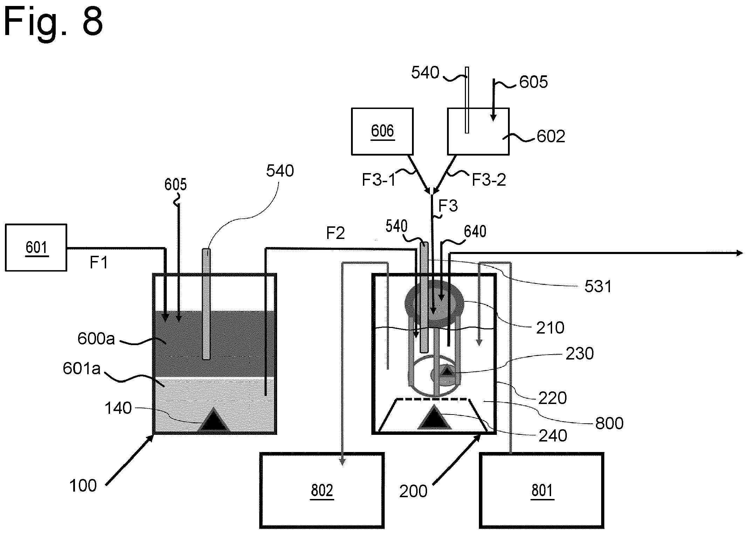

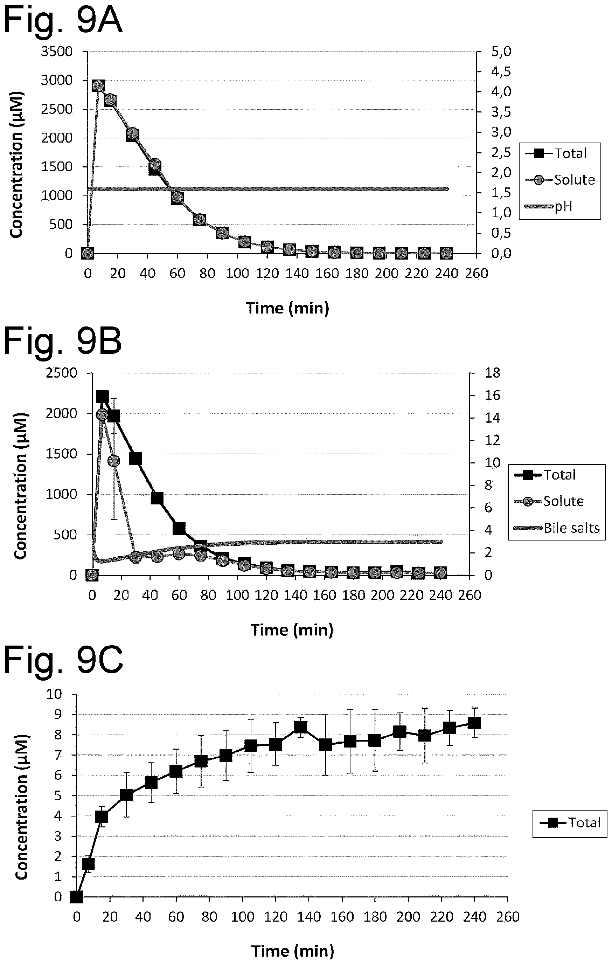

Figures 9A-10B are a series of graphs showing the results of an experiment, simulating fasted state and fed state conditions of the GIT, in which tests under fasted state conditions are performed in an experimental setup as shown in figure 1 and experiments under fed state conditions are performed as shown in figure 8.
Before the start of the fasted state experiments, 55 mL of simulated gastric juice was added to the stomach vessels, to simulate the rest volume of the stomach under fasted conditions, 30 mL of simulated duodenal juice was added to the inner vessel of the second compartment, and 300 mL of sink solution was added to the outer vessel of the second compartment. At the start of experiments, 696 mg indinavir sulfate was added to the stomach together with 250 mL of water During the experimental run the dynamics of the GIT were simulated through gastric secretion (flow rate F1) of simulated gastric fluid into the stomach, gastric emptying (flow rate F2; combination of mono-exponential emptying of the dose and emptying of the added gastric secretion) from stomach to the inside of the inner vessel, duodenal secretion (flow rate F3) from an exterior reservoir inside the inner vessel and duodenal emptying (flow rate F4 = F2 + F4) out of the inner vessel. As such, the volume of the stomach decreased monoexponentially in function of time whereas the volume of the inner vessel and the outer vessel remained constant. The pH of the content of the stomach was automatically controlled at a setpoint of pH 1,6. The pH of the duodenum (inner vessel) was kept constant at a value of 6,5 through active pH control and the secretion of duodenal fluids in the inner vessel. Active pH control was performed on the duodenal secretion, making it possible to change the buffering strength of the duodenal secretions in function of time. The pH of the outer vessel was set at a value of 6,5.
The content of the stomach reactor was homogenized through stirring at 300 rpm. The content of the inner and outer vessel was homogenized through stirring at 500 rpm. The temperature of the respective vessels was controlled at 37°C (Figure 1 for reference to experimental setup).
Each experimental run was followed for 240 min and samples were taken from the stomach vessel (first compartment), the duodenum (inner vessel) and sink (outer vessel) after 7, 15, 30, 45, 60, 75, 90, 105, 120, 135, 150, 165, 180, 195, 210, 225, and 240 min. In the samples taken from the stomach and duodenum the total and solute concentration of indinavir was determined. In the samples taken from the sink (outer vessel) the solute concentration of indinavir was determined.

Two sets of fed state experiments were performed. Before the start of each of the experiments, the same volumes and fluids were added to the stomach vessel and inner and outer vessel of the second compartment as during the fasted state experiments. At the start of the experiments simulating high stomach pH fed conditions, 696 mg indinavir sulfate was added to the stomach together with 400 mL of the nutritional drink Ensure plus and 250 mL of water. At the start of the low stomach pH fed experiments, 696 mg indinavir sulfate was added to the stomach together with 400 mL of carbohydrate solution (same caloric content as Ensure plus) and 250 mL of water. 15 min after the start of the experiment, simulating the lag phase for gastric emptying under fed state conditions, the dynamics of the fed state upper GIT were simulated through gastric secretion (flow rate F1) of simulated gastric fluids into the stomach, gastric emptying (flow rate F2; combination of emptying of the added gastric secretion and the flow rate needed to empty the ingested stomach fluids within 240 min) from the stomach to the inside of the inner vessel, duodenal secretion (constant flow rate F3 which is a combination of the dynamically varying flow rates of buffer solution 606 with flow rate F3-1 and simulated duodenal fluid 602 with flow rate F3-2, which are pumped from exterior reservoirs into the inner vessel). The ratios of the flow rates of F3-1 and F3-2 were selected to generate the dynamic bile salts, phospholipid, and enzyme concentrations in the duodenum as observed in vivo. Finally, the duodenum was emptied with a constant flow rate F6 (sum of gastric emptying and duodenal secretions). As such, the volume of the stomach emptied linearly in function of time whereas the volume of the inner and outer vessel remained constant. During the high stomach pH experiments the pH value of the stomach was controlled online and was subjected to a sigmoidal decrease from an initial value of 4,6 till a final value of 1,6 over 240 min, simulating the impact of a protein rich meal on the stomach pH. During the experiments under low stomach pH fed conditions the pH of the stomach was kept constant at a value of 1,6. During each fed state experiment the pH of the duodenum (inner vessel) was kept constant at 5,8 through a combination of active pH control and buffer secretion, and the pH of the sink (outer vessel) was set at a value of 5,8 (Figure 8).

The content of the stomach reactor was homogenized through stirring at 500 rpm. The content of the inner and outer vessel of the second compartment was homogenized through stirring at 500 rpm. The temperature of the respective vessels was controlled at 37°C.
Each experimental run was followed for 240 min and samples were taken from the stomach vessel (first compartment), the duodenum (inner vessel) and sink (outer vessel) after 22, 30, 45, 60, 75, 90, 105, 120, 135, 150, 165, 180, 195, 210, 225, and 240 min. In the samples taken from the stomach and duodenum the total and solute concentration of indinavir sulfate was determined. In the samples taken from the sink (outer vessel) the solute concentration of indinavir sulfate was determined.

All experimental runs were performed in biological triplicate and concentrations of indinavir sulfate in the different compartments of the DIAMOD were determined through UHPLC-UV.

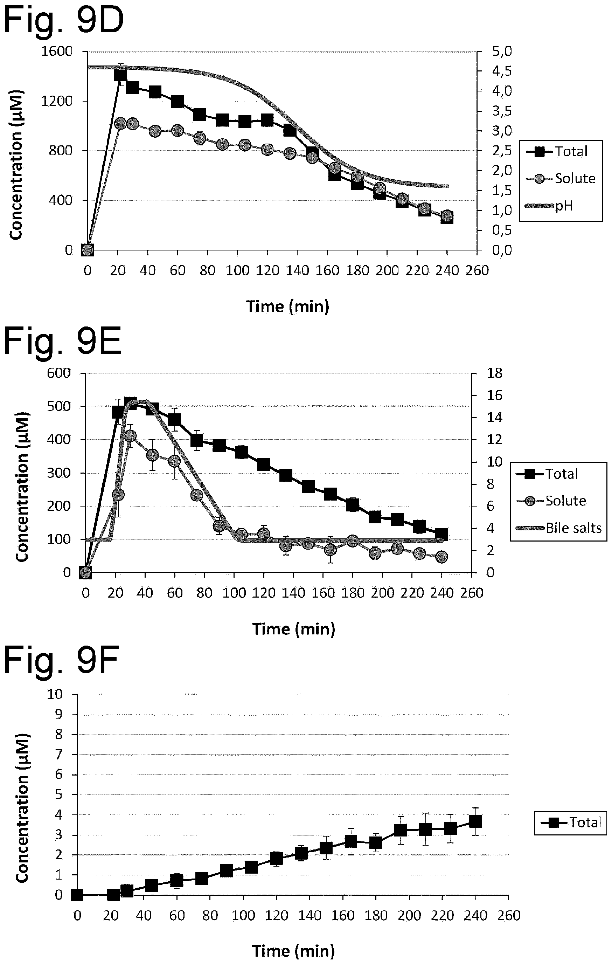

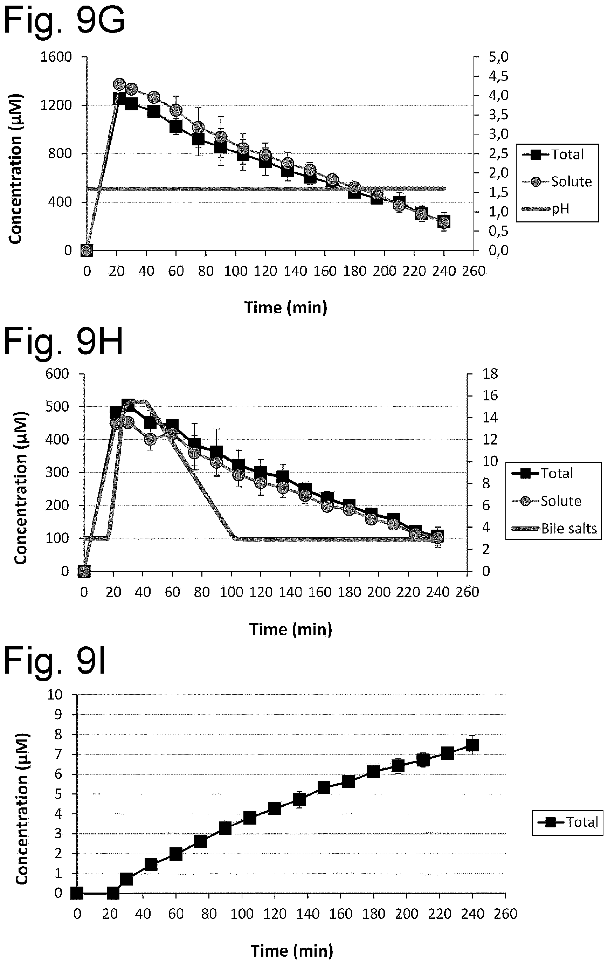

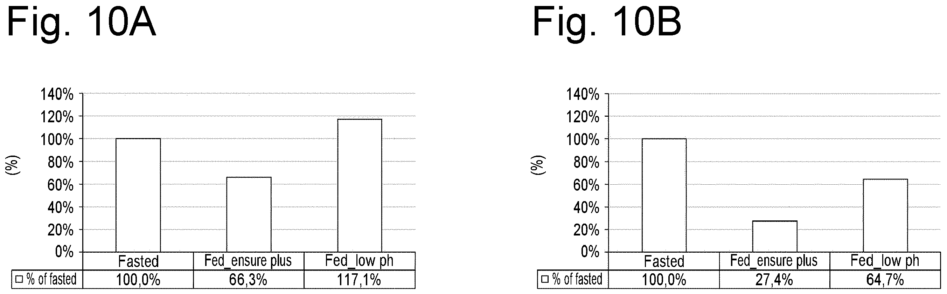

In the experiment, the capacity of the of the experimental setup to replicate the in vivo observed complex gastrointestinal behavior of indinavir sulfate under fasted and fed state conditions and the capacity to elucidate the important factors that contribute to the in vivo observed negative food effect is studied.
Indinavir is an HIV protease inhibitor and is a weak basic drug with pKa's of 3,7 and 5,9. Due to this feature, indinavir has a pH-dependent solubility, being highly soluble under acidic conditions but very poorly soluble at neutral pH environments. Yeh et al. (1998) demonstrated that indinavir sulfate was subjected to a substantial negative food effect when the compound was ingested together with a high caloric high fat meal as compared to ingestion of the drug under fasted state conditions. The authors attributed the occurrence of this negative food effect to the increased pH of the fed stomach probably resulting in the precipitation of indinavir in the stomach thereby reducing its small intestinal solute concentration, hence reducing its systemic exposure. Next to this, slower gastric emptying under fed state conditions as compared to fasted state conditions could have contributed to the observed effect. Carver et al. (1999) tested this hypothesis by dosing indinavir sulfate to fasted state individuals and individuals having ingested different types of isocaloric meals. The meals consisted of a high protein, high fat, and high carbohydrate meal which all had a different impact on the fed stomach pH upon ingestion. Being isocaloric, each meal resulted in the same delay in gastric emptying as compared to the fasted state condition. Whereas only the protein rich diet resulted in a substantial increase of the stomach pH, the ingestion of the high lipid and high carbohydrate meal resulted in comparable gastric pH values as present under fasted state conditions. Nevertheless, dosing of indinavir sulfate together with a high protein, high fat or high carbohydrate meal resulted in a decrease in indinavir bioavailability of 68%, 34%, or 45% as compared to dosing under fasted conditions. Considering the absence of any stomach pH effect by the high fat and high carbohydrate meals, these data indicated that the negative food effect for indinavir was not solely mediated by an increased stomach pH. Another important difference between fed and fasted state conditions is the high concentration of bile salts, phospholipids, and digestion products under fed conditions. These food-induced components can solubilize poorly aqueous soluble compounds through micellar entrapment. Notwithstanding the increase in solute drug, this micellar entrapment can result in a lower bio accessible concentration of indinavir resulting in a decreased absorption of the drug.
The results show that dosing of indinavir sulfate under fasted state conditions resulted in the complete dissolution of this compound in the stomach. This could be attributed to the low pH value of 1,6 of the stomach which was well below the pKa of indinavir namely 5,9. The total and solute concentration of indinavir decreased in function of time due to intragastric dilution of the stomach with gastric secretions and the emptying of the drug from the stomach through fast mono exponential stomach emptying (Figure 9A). During transition of the drug from the stomach to the duodenum a substantial pH shift occurred. The pH value of the duodenum was well above the pKa of indinavir which could have major implications on its solubility. During the initial phase of the experiments, high solute duodenal concentrations of indinavir were detected after which the concentration of solute indinavir became substantially lower than the total measured concentration indicating partial precipitation of indinavir in the near neutral pH environment of the duodenum (Figure 9B). The presence of high concentrations of solute indinavir in the duodenum during the initial phase of the experiments resulted in a high driving force for absorption which resulted in fast permeation of indinavir in the outer vessel (Figure 9C). During the fed state conditions with Ensure plus lower total concentrations of indinavir were present in the stomach as compared to the fasted state conditions. This was due to the higher dilution of the total ingested dose by the combined intake of Ensure plus and water, whereas only water was ingested under fasted state conditions. Comparison of the total and solute concentration of indinavir revealed that the elevated pH of the stomach did not result in the complete absence of dissolution of indinavir in the fed stomach. On the contrary, dosing of indinavir sulfate to the high pH stomach resulted in substantial dissolution of the drug. Interestingly, the data demonstrated that during the initial 150 min of the experiments indinavir was not completely dissolved in the stomach as was evident from the lower solute concentrations as compared to the total concentrations. After 160 min, indinavir became fully dissolved in the stomach and this was due to the lowering of the stomach pH in function of time. As such, both solid and solute indinavir were emptied from the stomach into the duodenum during the initial 150 min, whereas fully dissolved indinavir was provided to the duodenum between 160 and 240 min of the experimental run (Figure 9D). Indinavir demonstrated a complex behavior in the duodenum. Gastric emptying of indinavir from the stomach resulted in the presence of high concentrations of solute drug during the initial phases of the experiments. The comparable concentrations of total and solute indinavir demonstrated the near complete solubilization of indinavir by the high concentrations of bile salts present in the duodenum. Indeed, after 45 min the concentration of bile salts decreased in function of time in the duodenum, reaching basal fasted state levels after 105 min. The decrease in bile salts concentrations resulted in a decrease in the concentration of bile salt-solubilized indinavir in the duodenum as was revealed by the difference in between the concentration of total and solute indinavir at these time points. Since indinavir was not completely solubilized in the stomach during the first 160 min it appeared that the entrance of both solid and solute indinavir in the duodenum in the absence of high bile salt concentrations resulted in substantial duodenal precipitation of indinavir. Only after 160 min, the difference between the total and solute concentration of indinavir became smaller in the duodenum. This indicated a better solubility of indinavir in the duodenum after 160 min which was probably since indinavir was completely solubilized in the stomach at this time (Figure 9E). Notwithstanding the high concentrations of indinavir present in the duodenum during the initial 90 min, only minor permeation occurred. Substantial permeation from the duodenum only took place after bile salt levels dropped to basal fasted state levels (Figure 9F). These data indicated that micellar entrapment resulted in a near complete solubilization of indinavir in the fed state duodenum but a sharp decrease in the bio accessible fraction of the drug resulting in a low absorption of the drug.
Dosing of indinavir sulfate together with water and the carbohydrate solution resulted in the complete dissolution of the drug in the stomach as was demonstrated by the equal concentrations of total and solute of indinavir in the stomach. Indeed, during this condition the pH of the stomach was at a low value of 1,6, simulating the impact on the stomach pH upon ingestion of a carbohydrate solution. As such, a completely dissolved indinavir solution was provided to the duodenum by the stomach (Figure 9G). Indinavir was completely dissolved in the duodenum throughout the experiment. Whereas indinavir was solubilized during the initial duodenal phase by the high concentrations of bile salts present, no precipitation of indinavir occurred once the bile salts dropped to basal fasted state concentrations and this probably due to the complete solubilization of the drug in the stomach (Figure 9H). The high concentration of solute indinavir in the duodenum resulted in permeation of this compound throughout the experimental run. Since permeation is a concentration-driven process, higher duodenal concentrations of indinavir should be translated in a faster flux of indinavir from the inner vessel over the membrane into the outer vessel, i.e., a faster increase in the permeated concentration in function of time. Notwithstanding the very high concentrations of soluble drug during the initial duodenal phase as compared to the later stages, the concentration of permeated indinavir in function of time followed a linear trend. Hence, these results demonstrated the initial micellar entrapment of the drug by bile salts thereby increasing the solute concentrations but lowering the concentration of molecularly dissolved drug available for absorption (Figure 9I).
Finally, comparison of the AUC for solute concentrations of indinavir in the duodenum under the different dosing regimens clearly revealed that dosing under fasted state conditions resulted in high concentrations of solute drug in the duodenum in function of time. Dosing of indinavir sulfate under fed state conditions together with the carbohydrate solution did not decrease the AUC as compared to fasted state conditions. On the contrary the AUC was increased under this dosing regimen. This was due to the absence of any substantial precipitation of indinavir in the duodenum whereas duodenal precipitation occurred during fasted state experiments. The higher concentrations of total indinavir delivered to the duodenum and the higher pH of the duodenum under fasted conditions as compared to dosing of indinavir with a carbohydrate solution (fed state conditions) resulted in a higher driving force for precipitation. Since the pH of the fed stomach in this case was the same as during the fasted state experiment, it can be concluded that increased dilution of the indinavir dose by the larger stomach volumes under fed state conditions in combination with a slower gastric emptying did not result in lower concentrations of solute indinavir in the duodenum. On the contrary, during dosing under fed state conditions with elevated stomach pH, the AUC of solute concentration in the duodenum decreased substantially indicating that the increased stomach pH under fed conditions contributes to the lower concentrations of solute indinavir in the duodenum (Figure 10A). However, the minor effects on the solute concentration of indinavir in the duodenum during the runs under fed conditions could not solely explain the substantial negative food effect as observed in vivo. Indeed, comparing the AUC of the permeated concentration of indinavir in between fasted and fed conditions revealed a substantial effect on the permeated concentration (Figure 10B).

These data clearly indicated a substantial contribution of micellar entrapment during fed state conditions to the observed drastic negative food effect for indinavir sulfate as observed in vivo. The high concentrations of bile salts, phospholipids, and digestion products present under fed state conditions resulted in solubilization of indinavir in the duodenum thereby resulting in the high concentrations of solute indinavir in the duodenum during fed conditions. On the contrary, this solubilization of indinavir through micellar entrapment resulted in a substantial decrease in the free molecularly dissolved concentration of the drug thereby drastically decreasing the concentration available for absorption and hence the total concentration of indinavir permeated under fed state conditions as compared to fasted state conditions.
The system according to the invention allowed to study the negative food effect for indinavir sulfate thereby resulting in a strong in vitro-in vivo correlation. Furthermore, the system highlighted both the role of increased gastric pH, slower gastric emptying, and micellar entrapment of indinavir sulfate under fed state conditions as compared to fasted state conditions thereby resulting into mechanistic insights into the in vivo observed negative food effect.

Other alternatives and equivalent embodiments of the present invention are conceivable within the idea of the invention, as will be clear to the person skilled in the art. The scope of the invention is limited only by the appended claims.

## Claims

1. A method of simulating the gastrointestinal dissolution and intestinal permeation of a substance, comprising the steps of:
a) providing a dynamic gastrointestinal tract simulation system comprising at least two consecutive compartments of which a first compartment simulates at least part of the stomach and a second compartment simulates at least part of the small intestine, said second compartment comprising an outer vessel and an inner vessel mounted inside the outer vessel, said inner vessel having a wall comprising a grid structure and a semi-permeable membrane mounted around said grid structure;
b) introducing the substance in the first compartment;
c) operating the gastrointestinal tract simulation system, further comprising a fluid transfer system for transferring fluids into and from said at least two consecutive compartments, to transfer the substance from the first compartment into said inner vessel and to allow permeation of said substance in a dissolved state through the membrane; and
d) sampling the fluid of the first compartment and of inner the inner vessel of the second compartment to assess the dissolution of said substance and/or sampling fluid from the outer vessel of the second compartment to assess the permeation of said substance through the membrane.

2. The method according to claim 1, wherein the gastrointestinal tract simulation system is operated fully dynamic.

3. The method according to any one of the preceding claims, wherein step a) comprises the substeps of:
al) providing an empty gastrointestinal tract simulation system comprising at least two consecutive empty compartments, and
a2) filling said empty compartments with fluids simulating the physiological fluids of the gastrointestinal tract.

4. The method according to any one of the preceding claims, wherein the gastrointestinal tract simulation system further comprises at least one stirring system for stirring the contents of the inner and/or outer vessel, and
the gastrointestinal tract simulation system is further operated to mix the contents of the inner vessel and/or the contents of the outer vessel by stirring the contents of the respective vessel.

5. The method according to any one of the preceding claims, wherein said substance is comprising a pharmaceutical substance,
wherein said pharmaceutical substance is selected from the group consisting of drugs classified according to the Biopharmaceutics Classification System as belonging to Class I, Class II, Class III, or Class IV.

6. The method according to claim 5 wherein said pharmaceutical substance is selected from the classes of pharmaceutical compounds that have a high permeability namely selected from the group of drugs classified according to the Biopharmaceutics Classification System as belonging to Class I or Class II.

7. The method according to any one of the preceding claims, wherein the gastrointestinal tract simulation system is operated to transfer, at pre-determined rates:
gastric secretion fluid from a first reservoir to the first compartment;
simulated gastric fluid from the first compartment to the inner vessel of a second compartment;
duodenal secretion fluid from a second reservoir to the inner vessel of the second compartment; and
simulated duodenal fluid from the inner vessel of the second compartment to a third reservoir.

8. The method according to claim 7, wherein the gastrointestinal tract simulation system is further operated to transfer, at pre-determined rates:
0-50 vol%/min fresh sink solution from a fresh sink reservoir to the outer vessel of the second compartment, and
0-50 vol%/min waste sink from the outer vessel of the second compartment to a waste sink reservoir.

9. The method according to any one of the preceding claims, further comprising operating the gastrointestinal tract simulation system so as to control, in each of the compartments, one or more or all of the following parameters: liquid flow, temperature, pH, ionic strength, head space, stirring, pressure, liquid volume, dissolved oxygen, redox potential, each in accordance with predetermined values, ranges or trajectories.

10. The method according to any one of the preceding claims, wherein a plurality of said gastrointestinal tract simulation systems are operated simultaneously and identically, and wherein one or more of said gastrointestinal tract simulation systems contain a substance and one or more of the gastrointestinal tract simulation systems do not contain a test sample.

11. The method according to any one of the preceding claims, wherein each compartment comprises a vessel having an open top surrounded by a peripheral edge portion and a lid system configured to be placed onto the peripheral edge portion and to form a seal between the lid system and the vessel, wherein the lid system comprises a body with a plurality of passageways extending through the body and providing access to the interior of the vessel, said plurality of passageways comprising first passageways configured for receiving fluid transfer tubes and second passageways configured for mounting at least one sensor component.

12. The method according to any one of the preceding claims, wherein the fluid transfer system comprises a plurality of peristaltic pumps for pumping fluid via fluid transfer tubes into and from said at least two consecutive compartments.

13. The method according to any one of the preceding claims, wherein the gastrointestinal tract simulation system comprises at least three consecutive compartments of which a third compartment simulates the jejunum,
wherein the gastrointestinal tract simulation system comprises at least four consecutive compartments of which a fourth compartment simulates the ileum, and/or
wherein the gastrointestinal tract simulation system further comprises a compartment simulating the average conditions of the combined consecutive compartments.

14. A gastrointestinal tract simulation system for simulating the gastrointestinal dissolution and intestinal permeation of substances, comprising at least two consecutive compartments of which a first compartment simulates the stomach and a second compartment simulates the duodenum, said second compartment comprising an outer vessel and an inner vessel mounted inside the outer vessel, said inner vessel having a wall comprising a grid structure and a semi-permeable membrane mounted around said grid structure.

15. Use of the system according to claim 14 to simulate the gastrointestinal dissolution and intestinal permeation of a substance.
